# EUROPEAN PATENT SPECIFICATION

(11) **EP 3 751 287 B1**
(45) Date of publication and mention of the grant of the patent: **14.02.2024**
(21) Application number: 20175994.1
(22) Date of filing: 22.05.2020
(51) Int. Cl.: G01N 35/00

(54) **TEST TUBE LABELING APPARATUS CAPABLE OF SEQUENTIAL LABELING AND RECORDING MEDIUM STORING PROGRAM FOR OPERATING THE SAME**
ZUM SEQUENZIELLEN ETIKETTIEREN FÄHIGE PRÜFRÖHRCHENETIKETTIERVORRICHTUNG UND AUFZEICHNUNGSPROGRAMM ZUR SPEICHERUNG EINES PROGRAMMS ZUM BETRIEB DAVON
APPAREIL D'ÉTIQUETAGE DE TUBES À ESSAI POUVANT EFFECTUER UN ÉTIQUETAGE SÉQUENTIEL ET SUPPORT D'ENREGISTREMENT STOCKANT UN PROGRAMME POUR LE FAIRE FONCTIONNER

(30) Priority: 10.06.2019 KR 20190068244
(43) Date of publication of application: 16.12.2020
(73) Proprietor: Energium Co., Ltd., Gyeonggi-do, 15115 (KR)
(72) Inventor: KANG, Junghun, 08504 Seoul (KR); LEE, Kanghee, 08504 Seoul (KR); KIM, Yong Won, 08504 Seoul (KR); GRIFFITH, Ronald Eric, 08504 Seoul (KR)
(74) Representative: Tiburzi, Andrea

(56) References cited:
- JP-A- 2010 085 289
- US-A- 5 688 361

## Description

### BACKGROUND

### Field

The disclosure relates to an apparatus for sequentially attaching labels to test tubes in accordance with priorities based on customer's information and discharging the label-attached test tubes in order, and also relates to a recording medium storing a program for operating the apparatus.

### Description of Related Art

Test tubes are used to store a great variety of samples such as a blood sample, an animal sample, a plant sample, and a chemical sample. A label is attached to such a test tube to indicate sample information.

Manually labeling a large number of test tubes requires a significant amount of work. In addition, such manual labeling may often degrade a labeling quality or cause wrong labeling.

In order to reduce the burden of work and improve the accuracy and quality of labeling, an automated apparatus may be used. However, a conventional labeling apparatus is still inconvenient because of requiring pre-alignment of test tubes to be supplied for labeling. Moreover, preparation of test tubes and labels according to a predetermined inspection order requires a separate manual work.

As such, in the conventional labeling apparatus, there is a problem that an operator manually prepares test tubes by determining an order according to customer's sample information.

In case of another conventional labeling apparatus that attaches labels with customer's sample information to test tubes inserted at random, there is a problem that a test tube is not discharged in consideration of an inspection order and a plurality of test tubes are accumulated in a storage unit.

Accordingly, there is a need to provide an apparatus for sequentially attaching labels to test tubes in accordance with priorities based on customer's information and discharging the label-attached test tubes in order.

JP 2010 085289 A discloses a device for preparing test tubes, wherein labels with bar codes are printed.

US 5 688 361 A discloses an automatic vessel supplying and labeling apparatus.

### SUMMARY

Various embodiments of the disclosure provide a test tube labeling apparatus capable of sequential labeling and a recording medium storing a program for operating the same.

According to the invention, provided is a test tube labeling apparatus according to claim 1.

According to the invention, provided is a computer-readable recording medium in which a program for executing operations of attaching a label to a test tube in the above test tube labeling apparatus is recorded according to claim 4.

The test tube labeling apparatus and the recording medium according to various embodiments of the disclosure can automatically and sequentially discharge the labeled test tubes in consideration of the priorities determined in accordance with the inspection order based on the requirements of a customer such as a hospital.

Further, the test tube labeling apparatus and the recording medium according to various embodiments of the disclosure can confirm that one test tube having a particular priority has been completely discharged from the test tube storage unit, and then perform labeling and discharging of the next test tube having the next priority. This can prevent the labeled test tubes from being accumulated unintentionally in the storage unit or being discharged out of order from the storage unit.

### BRIEF DESCRIPTION OF THE DRAWINGS

The above and other aspects, features and advantages of certain embodiments of the disclosure will be more apparent from the following detailed description, taken in conjunction with the accompanying drawings.
FIG. 1 is a view showing the configuration of a test tube labeling apparatus according to various embodiments of the disclosure.
FIG. 2 is a block diagram showing the configuration of a test tube labeling apparatus according to various embodiments of the disclosure.
FIG. 3 is a flowchart showing a program for operating a test tube labeling apparatus according to various embodiments of the disclosure.

### DETAILED DESCRIPTION

Hereinafter, various embodiments of the disclosure will be described in detail with reference to the accompanying drawings. The embodiments are not intended to limit the disclosure and should be understood to include various modifications, equivalents, and/or alternatives to corresponding embodiments. In the drawings, similar reference numbers are used to indicate similar elements.

In the disclosure, the terms such as "comprise", "include", and "have" denote the presence of stated elements, components, operations, functions, features, and the like, but do not exclude the presence of or a possibility of addition of one or more other elements, components, operations, functions, features, and the like.

In the disclosure, the expressions "A or B", "at least one of A and/or B", "one or more of A and/or B", and the like may include all possible combinations of items listed together. For example, "at least one of A or B" may indicate all of (1) including at least one A, (2) including at least one B, and 3) including both of at least one A and at least one B.

Terms used herein may be merely to describe a certain embodiment, and may not be intended to limit the scope of other embodiments. The singular expressions may include plural expressions unless the context clearly dictates otherwise. Terms used herein, including technical or scientific terms, may have the same meaning as commonly understood by those skilled in the art. Some terms defined in a normal dictionary may be interpreted as having the same or similar meaning as the contextual meanings in the related art. Certain terms are not to be construed as an ideal or overly formal sense unless expressly defined to the contrary herein. In some cases, the terms defined herein cannot be construed to exclude embodiments of the present disclosure.

A test tube labeling apparatus according to various embodiments of the disclosure may indicate, for example, a tube alignment device for a blood-collecting tube, an automatic supply device for a blood-collecting tube for a labeler, a test tube supply device, a test tube preparation device, a test tube transport device, a labeling device, a tube container labeling device, or any apparatus including them.

Now, the test tube labeling apparatus according to various embodiments will be described with reference to the accompanying drawings.

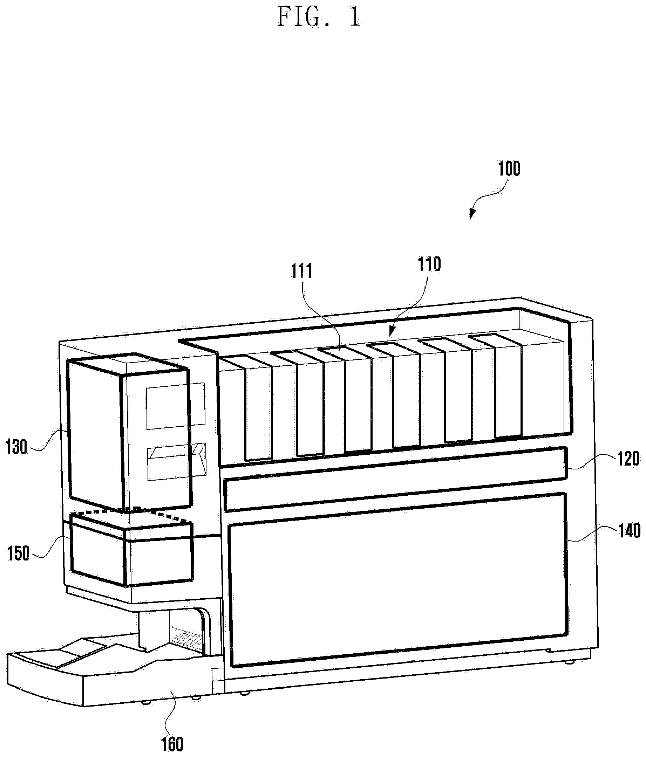

FIG. 1 is a view showing the configuration of a test tube labeling apparatus 100 according to various embodiments of the disclosure. The names of elements according to various embodiments are not standardized and may vary depending on the function of each element. For example, a controller 130 may be referred to as another name such as a processor.

As shown in FIG. 1, in the test tube labeling apparatus 100 according to various embodiments, a cassette 111 may have an inlet formed on a front portion to allow a plurality of test tubes to be stacked in a horizontal state, and an outlet formed on a lower portion to discharge the stacked test tubes one by one. Such cassettes 111 may constitute a cassette unit 110. The cassette 111 may discharge the test tubes, supplied at random, one by one with rotation. The test tubes discharged one by one through the outlet of the cassette 111 may be located in a transporting unit 120.

The transporting unit 120 performs a function of transporting the test tube. The transporting unit 120 has a discharge port connected to a label attaching unit 150 and allowing the test tubes to be discharged one by one. The transporting unit 120 may be located below the cassette unit 110 as shown in FIG. 1, but the position of the transporting unit 120 is not limited thereto. Alternatively, any other position where the test tube can be supplied from the cassette unit 110 is possible. The transporting unit 120 may be implemented, for example, in the form of a conveyor belt to transport the test tube.

The test tube labeling apparatus 100 according to various embodiments may include a memory (not shown) as a storage device. The memory (not shown) of the test tube labeling apparatus 100 may store information to be printed on a label to be attached to the test tube, and information about the operation of the test tube labeling apparatus 100.

The information to be printed on the test tube label includes basic information (i.e. identification information) and inspection information of an inspection target. Such information is provided in the form of a barcode. Also, such information is provided to a label printing unit 140 for printing by a controller 130.

The information about the operation of the test tube labeling apparatus 100 may include information about inspection priorities based on requirements of a customer such as a hospital, and information about unique numbers of the individual cassettes 111.

The inspection priority information may be information indicating, for example, that an examination for a specific disease should be performed first.

The individual cassette number information may be information specifying the unique numbers assigned to the individual cassettes 111, for example, when blood-collecting information for several diseases is required. That is, based on the individual cassette number information, a blood-collecting operation or examination for a specific disease can be performed for a specific test tube supplied to the cassette 111 having the corresponding number.

The test tube labeling apparatus 100 according to various embodiments may include a communication module (not shown) for transmitting and receiving information to and from an external device (e.g., a central computer of a hospital or a personal computer of a doctor). The transmission/reception of information through the communication module may be performed using a wired or wireless communication network, and the received information may be stored in the memory of the test tube labeling apparatus 100.

In addition to a method of using the wired or wireless communication network, other methods may be used for storing necessary information in the memory of the test tube labeling apparatus 100. For example, information may be provided to the memory of the test tube labeling apparatus 100 through a removable storage medium. In another example, when the test tube labeling apparatus 100 is manufactured, default information may be stored in advance in the memory of the test tube labeling apparatus 100. In still another example, when the test tube labeling apparatus 100 is installed, particular information reflecting customer's requirements may be stored in the memory of the test tube labeling apparatus 100.

The controller 130 according to various embodiments may be operatively connected to the cassette unit 110 composed of the plurality of cassettes 111, the transporting unit 120, the label printing unit 140, the label attaching unit 150, and a test tube storage unit 160.

The function or operation of the controller 130 is as follows.

The controller 130 may access a database in the memory of the test tube labeling apparatus 100 or an external device and then, based on information obtained from the database, control the cassette unit 110 including the plurality of cassettes 111 to discharge the test tubes one by one.

Also, in response to a labeling request that satisfies the inspection priority information obtained from the database, the controller 130 may control the transporting unit 120 to sequentially transport the test tubes, discharged one by one from the cassette unit 110, to the label attaching unit 150.

Also, in response to a printing request, the controller 130 may control the label printing unit 140 to print information including the identification information and the inspection information of the inspection target on labels to be attached to the test tubes sequentially transported to the label attaching unit 150.

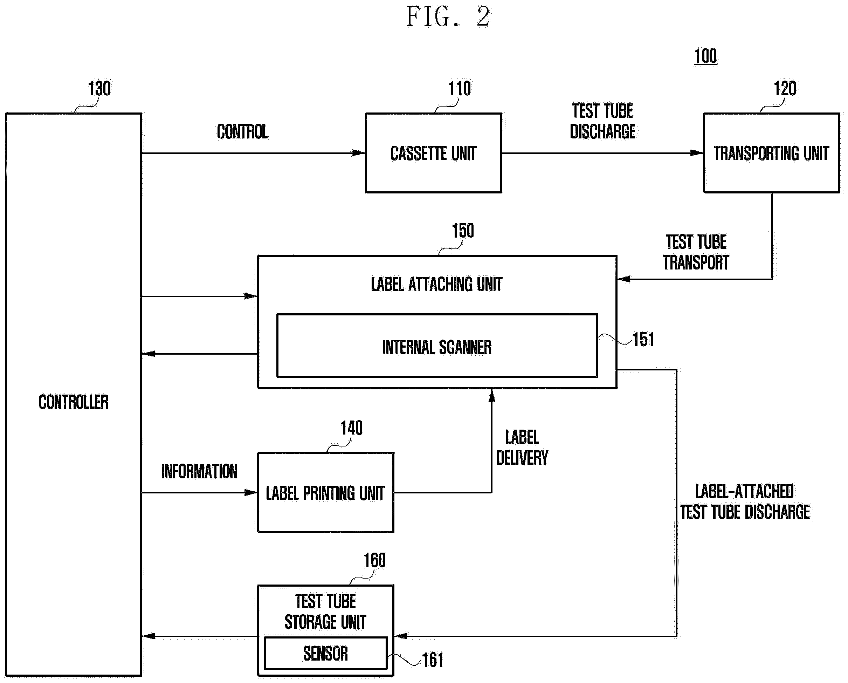

In addition, the controller 130 may control an internal scanner 151 (see FIG. 2) of the label attaching unit 150 to determine whether the information printed on each label is identical to the information stored in the database. If there is no error in the printed label information as a result of determination, the controller 130 may prepare to discharge the labeled test tube to the test tube storage unit 160.

In addition, the controller 130 controls a sensor 161 (see FIG. 2) of the test tube storage unit 160 to detect the presence or absence of each test tube in the test tube storage unit 160. When there is no test tube in the test tube storage unit 160, the controller 130 may control the label attaching unit 150 to discharge the labeled test tube to the test tube storage unit 160.

According to some embodiment, the controller 130 may continuously control the test tube labeling apparatus 100, from the cassette unit 110 to the test tube storage unit 160, with respect to one test tube selected based on the inspection priority. Whenever each test tube is completely discharged from the test tube storage unit 160, the controller 130 may control again the test tube labeling apparatus 100 to repeatedly operate with respect to the next test tube.

The label printing unit 140 receives a label from a supply roller (not shown) on which the label is wound. In addition, under the control of the controller 130 that accesses the database in the memory of the test tube labeling apparatus 100 or the external device, the label printing unit 140 prints information obtained from the database on the supplied label. The printed information may include identification information of an inspection target (e.g., a patient) and inspection information (e.g., an inspection item, a test tube type, a blood-collecting amount) of the inspection target.

The label attaching unit 150 attaches the label, supplied from the label printing unit 140, to the test tube supplied through the transporting unit 120. Thereafter, the label attaching unit 150 discharges the labeled (i.e., label-attached) test tube to the test tube storage unit 160. The test tube storage unit 160 is a space for storing the test tube discharged from the label attaching unit 150. The test tube storage unit 160 includes the sensor 161 capable of detecting the presence or absence of an object (i.e., the test tube).

As the sensor 161 of the test tube storage unit 160, an infrared sensor may be used for example. Alternatively, a pressure sensor capable of weight sensing or a contact sensor capable of sensing a physical contact may be used. The sensor 161 detects whether a test tube exists inside the test tube storage unit 160, so that the controller 130 can determine whether a test tube should be discharged from the label attaching unit 150. That is, only when the detecting result indicates that there is no test tube, the controller 130 may control the label attaching unit 150 to discharge one test tube.

The test tube labeling apparatus 100 shown in FIG. 1 may further include a display unit (not shown) capable of displaying label information and/or customer information on a screen. The display unit may be provided with an auxiliary input device such as a scanner capable of scanning a barcode of a label and thereby obtaining the label information.

FIG. 2 is a block diagram showing the configuration of a test tube labeling apparatus according to various embodiments of the disclosure.

For example, in order to initiate an examination through blood-collecting in a hospital, a blood-collecting worker may supply test tubes, which are not labeled, to the cassette unit 110 including the plurality of cassettes 111. Also, through information stored in the memory of the test tube labeling apparatus 100 and/or an external device, the blood-collecting worker may request an unlabeled test tube supply. In response to the test tube supply request, the controller 130 may control the cassette unit 110 to discharge test tubes one by one from the plurality of cassettes 111.

In order to attach the label to the test tube, the controller 130 may control the transporting unit 120 to transport the test tube, discharged from the cassette unit 110 including the plurality of cassettes 111, to the label attaching unit 150.

The controller 130 allows the test tubes to be sequentially supplied based on inspection priority information obtained by accessing the database in the memory of the test tube labeling apparatus 100 and/or the external device. In addition, the controller 130 may control the label printing unit 140 to print information including identification information and inspection information of an inspection target, obtained from the database, on the label. In addition, the controller 130 may control the label attaching unit 150 to attach the printed label to the test tube.

The test tube storage unit 160 includes the sensor 161 for detecting an inflow and an outflow of each test tube in the test tube storage unit 160. In response to receiving an inflow detection signal and an outflow detection signal for one test tube having a particular priority from the sensor 161, the controller 130 enables a next test tube having a next priority to be supplied to the label attaching unit 150 and also enable a next label to be printed with information including the identification information and the inspection information of the inspection target corresponding to the next priority and then attached to the next test tube.

In addition, based on the detection signals received from the sensor 161, the controller 130 may control the label attaching unit 150 to discharge the next label-attached test tube only when there is no test tube in the test tube storage unit 160.

Hereinafter, a recording medium in which a program for operating a test tube labeling apparatus according to various embodiments is recorded will be described with reference to the accompanying drawings.

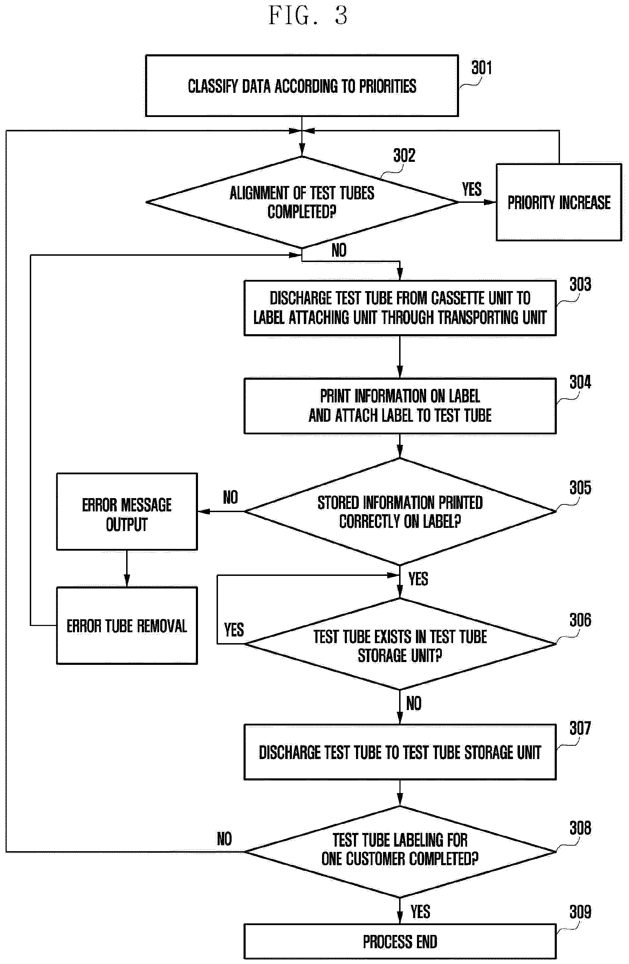

FIG. 3 is a flowchart showing a program for operating a test tube labeling apparatus according to various embodiments of the disclosure.

A computer-readable recording medium for recording or storing the program for operating the test tube labeling apparatus 100 according to various embodiments may be a removable storage medium. The type of the recording medium is not necessarily limited to the removable storage medium as long as the controller 130 of the test tube labeling apparatus 100 can access and control the recording medium.

The recording medium in which the program for executing operations of the test tube labeling apparatus 100, based on inspection priority information, is recorded may be provided together when the test tube labeling apparatus 100 is provided.

The operations based on the inspection priority information may include operating the controller 130 of the test tube labeling apparatus 100 to access a database that stores identification information, inspection information, and inspection priority information of an inspection target.

In addition, the operations may include operating the controller 130 of the test tube labeling apparatus 100 to control the cassette unit 110 and the transporting unit 120 of the test tube labeling apparatus 100 to sequentially discharge and transport the test tubes to the label attaching unit 150 of the test tube labeling apparatus 100, based on the inspection priority information obtained from the database. Also, the operations may include operating the controller 130 of the test tube labeling apparatus 100 to control the label printing unit 140 of the test tube labeling apparatus 100 to print information including the identification information and the inspection information of the inspection target on the labels to be attached to the test tubes sequentially transported to the label attaching unit 150.

In addition, the operations may include operating the controller 130 of the test tube labeling apparatus 100 to control the cassette unit 110 including the plurality of cassettes 111 to discharge the plurality of test tubes one by one. Also, the operations may include operating the controller 130 of the test tube labeling apparatus 100 to control the label attaching unit 150 to attach the printed label to the test tube, and control the test tube storage unit 160 to accommodate the label-attached test tube.

The program may be installed in a central computer of a hospital or a personal computer of a doctor. The program may be provided to the central computer of the hospital and/or the personal computer of the doctor through a removable storage medium. Also, the program may be installed in the memory of the test tube labeling apparatus 100 or operated in the test tube labeling apparatus 100 through a removable storage medium.

The identification information, inspection information, and inspection priority information of an inspection target for the operation of the test tube labeling apparatus 100 may be stored in the memory of the test tube labeling apparatus 100 and used through the controller 130, or stored in an external device and used through the controller 130. Alternatively, both methods may be used.

Referring to FIG. 3, the controller 130 of the test tube labeling apparatus 100 may classify data according to priorities based on the requirements of a customer such as a hospital (operation 301). For example, the controller 130 may be configured to classify data related to identification information, inspection information, and inspection priority information of an inspection target. The controller 130 may acquire information necessary for classification by accessing the database.

According to an embodiment, the controller 130 may be configured to check whether the test tube alignment by priorities is completed (operation 302). That is, after classifying data according to priorities, the controller 130 may determine whether printing of a label for one customer is completed for sequential labeling. In this case, when printing for one customer has not been completed, the controller 130 may determine that alignment of test tubes according to priorities is not completed.

The controller 130 may be configured to discharge the test tube from the cassette unit 110 to the label attaching unit 150 through the transporting unit 120 (operation 303). According to another embodiment, without performing the operation 302, the controller 130 may immediately perform the operation 303 after the operation 301. This may be, for example, a case where the test tube labeling is performed for the first time by booting or rebooting of the test tube labeling apparatus 100. That is, this may correspond to a case where any label printing has been not performed yet.

At the operation 302, the controller 130 may check whether the alignment of test tubes is completed in order to discharge the labeled test tubes one by one in accordance with priorities. That is, after confirming that the alignment of test tubes by priorities is completed, the controller 130 may proceed to labeling a test tube of the next priority. The operation 302 may be an operation of checking whether the test tubes have been successfully labeled according to a current priority and discharged.

According to an embodiment, the controller 130 may repeat the process shown in FIG. 3 when the test tube labeling for the first-priority inspection target is not completed. For example, the controller 130 may be configured to repeat the operations from 302 to 308 when it is determined at operation 308 that the test tube labeling for one customer (e.g., the first-priority inspection target) is not completed.

According to another embodiment, the controller 130 may be configured to perform the test tube labeling for the second-priority inspection target when the test tube labeling for the first-priority inspection target is completed. In this case, based on determining that the test tube labeling for one customer (e.g., the first-priority inspection target) is completed, the controller 130 may terminate the process for the first-priority inspection target at operation 309. Then, in order to proceed with a new process for an inspection target of the second priority, the controller 130 may return to the operation 302 for checking whether the alignment of test tubes is completed, and may also increase the priority to the second priority. The checking operation 302 may be what the controller 130 confirms that the process for a part of the entire data received from the database is completed.

If it is determined at the operation 308 that the test tube labeling for the first-priority inspection target has not been completed, the controller 130 may perform the operation 302 again. In this case, because the test tube alignment for the first-priority inspection target is not completed, the controller 130 may perform the operation 303 without increasing the priority to the second priority. According to another embodiment, after the test tube labeling for the first-priority inspection target is completed, the test tube labeling for the second-priority inspection target may be performed based on the data classified according to priorities. In this case, the controller 130 may determine at the operation 302 that the test tube alignment for the first-priority inspection target is completed, and may also increase the priority in order to perform a new process for the test tube labeling for the second-priority inspection target.

The labeling process of the test tube labeling apparatus 100 may be performed in accordance with the flowchart of FIG. 3. When the test tube labeling for all inspection targets according to priorities is completed, the process may be ended at the operation 309. In the flowchart of FIG. 3, not all operations should be sequentially performed, and some operations may be changed or performed in parallel.

According to various embodiments, the test tube labeling apparatus 100 discharges test tubes one by one from the cassette unit 110 including a plurality of cassettes 111 in accordance with priorities based on the requirements of a customer (e.g., a hospital) (operation 301), and transports the test tubes to the label attaching unit 150 through the transporting unit 120 (operation 303). In addition, the test tube labeling apparatus 100 obtains information from the database in the memory and/or the external device, prints the obtained information on a label in the label printing unit 140, and attaches the printed label to the test tube in the label attaching unit 150 (operation 304). Thereafter, the test tube labeling apparatus 100 discharges the label-attached test tube from the label attaching unit 150 to the test tube storage unit 160 (operation 307).

According to some embodiments, between the operations 304 and 307, the test tube labeling apparatus 100 may determine at operation 305 whether the obtained (i.e., stored) information is printed correctly on the label. That is, the controller 130 may control the internal scanner 151 of the label attaching unit 150 to determine whether the information printed on the label is identical to the information stored in the database. If there is no error in the printed label information, the controller 130 may prepare to discharge the labeled test tube to the test tube storage unit 160. If there is any error in the printed label information, the controller 130 may output an error message, remove the error tube, and repeat the operations 303 to 305.

According to some embodiments, between the operations 304 and 307, the test tube labeling apparatus 100 determines at operation 306 whether a test tube exists in the test tube storage unit 160. That is, the controller 130 controls the sensor 161 of the test tube storage unit 160 to detect the presence or absence of any test tube in the test tube storage unit 160.

The controller 130 performs the operation 306 by receiving an inflow detection signal and an outflow detection signal from the sensor 161 in the test tube storage unit 160. When there is any test tube in the test tube storage unit 160, the controller 130 may repeat the operation 306 periodically.

When it is determined at the operation 306 that there is no test tube in the test tube storage unit 160, the controller 130 may control the label attaching unit 150 to discharge the labeled test tube to the test tube storage unit 160 at the operation 307 and then determine at the operation 308 whether the test tube labeling for one customer is completed.

Thereafter, the test tube labeling apparatus 100 may end the process for one inspection target at the operation 309 or repeat the above-described operations 302 to 308.

While the disclosure has been particularly shown and described with reference to exemplary embodiments thereof, it will be understood by those skilled in the art that various changes in form and details may be made therein without departing from the scope of the subject matter as defined by the appended claims.

## Claims

1. A test tube labeling apparatus (100) comprising:
a cassette unit (110) including a plurality of cassettes (111), each cassette (111) configured to accommodate a plurality of test tubes and to include an outlet formed on a lower portion to discharge the plurality of test tubes one by one;
a transporting unit (120) receiving a test tube from the cassette unit (110) and transporting the test tube;
a label printing unit (140) printing information on a label to be attached to the test tube;
a label attaching unit (150) receiving the test tube from the transporting unit (120), receiving the information-printed label from the label printing unit (140), and attaching the information-printed label to the test tube;
a test tube storage unit (160) accommodating the label-attached test tube received from the label attaching unit (150); and
a controller (130) operatively connected to the cassette unit (110), the transporting unit (120), the label printing unit (140), the label attaching unit (150), and the test tube storage unit (160),
wherein the label attaching unit (150) includes an internal scanner (151) scanning a barcode information of the label printed from the label printed unit (140),
wherein the controller (130) is configured to:
access a database that stores identification information, inspection information, and inspection priority information of an inspection target,
control the cassette unit (110) and the transporting unit (120) to sequentially discharge and transport the test tubes to the label attaching unit (150), based on the inspection priority information obtained from the database, and
control the label printing unit (140) to print information including the identification information and the inspection information of the inspection target on the labels to be attached to the test tubes sequentially transported to the label attaching unit (150), control the label attaching unit (150) to attach the printed labels to the test tubes,
control the internal scanner (151) to scan the barcode information of the label attached to the test tube in order to determine whether the barcode information of the label attached to the test tube by the label attaching unit (150) matches a barcode information stored in the database, generate an error message based on determining that the barcode information of the label attached to the test tube does not match the barcode information stored in the database, and repeat the operation of discharging and transporting a test tube to the label attaching unit (150), printing information by the label printing unit (140) and attaching the printed label by the label attaching unit (150),
wherein the test tube storage unit (160) includes a sensor (161) for detecting an inflow and an outflow of each test tube in the test tube storage unit (160), and
wherein in response to receiving an inflow detection signal and an outflow detection signal for one test tube having a particular priority from the sensor (161), the controller (130) enables a next test tube having a next priority to be supplied to the label attaching unit (150) and also enables a next label to be printed with information including the identification information and the inspection information of the inspection target corresponding to the next priority and enables the next label to be attached to the next test tube.

2. The test tube labeling apparatus (100) of claim 1, further comprising:
a storage device including the database.

3. The test tube labeling apparatus (100) of claim 1, further comprising:
a communication module capable of communicating with an external electronic device,
wherein the database is stored in the external electronic device, and the controller (130) accesses the database through the communication module.

4. A computer-readable recording medium in which a program for executing operations of attaching a label to a test tube in the test tube labeling apparatus (100) set forth in claim 1 is recorded, the program causing, when executed, the controller (130) of the test tube labeling apparatus (100) to:
access the database that stores identification information, inspection information, and inspection priority information of an inspection target,
control the cassette unit (110) and the transporting unit (120) of the test tube labeling apparatus (100) to sequentially discharge and transport the test tubes to the label attaching unit (150) of the test tube labeling apparatus (100), based on the inspection priority information obtained from the database, and
control the label printing unit (140) of the test tube labeling apparatus (100) to print information including the identification information and the inspection information of the inspection target on the labels to be attached to the test tubes sequentially transported to the label attaching unit (150),
control the label attaching unit (150) to attach the printed labels to the test tubes,
control the internal scanner (151) to scan the barcode information of the label attached to the test tube in order to determine whether the barcode information of the label attached to the test tube by the label attaching unit (150) matches a barcode information stored in the database,
generate an error message based on determining that the barcode information of the label attached to the test tube does not match the barcode information stored in the database, and repeat the operation of discharging and transporting a test tube to the label attaching unit (150), printing information by the label printing unit (140) and attaching the printed label by the label attaching unit (150),
wherein the program further causes, when executed, the controller (130) of the test tube labeling apparatus (100) to:
in response to receiving an inflow detection signal and an outflow detection signal for one test tube having a particular priority from the sensor (161) that is installed in a test tube storage unit (160) of the test tube labeling apparatus (100) to detect an inflow and an outflow of each test tube in the test tube storage unit (160), enable a next test tube having a next priority to be supplied to the label attaching unit (150), and
enable a next label to be printed with information including the identification information and the inspection information of the inspection target corresponding to the next priority and enable the next label to be attached to the next test tube.

## Patentansprüche

1. Vorrichtung zum Etikettieren von Reagenzgläsern (100), umfassend: eine Kassetteneinheit (110) mit mehreren Kassetten (111), wobei jede Kassette (111) so konfiguriert ist, dass sie mehrere Reagenzgläser aufnimmt und einen an einem unteren Abschnitt ausgebildeten Auslass aufweist, um die mehreren Reagenzgläser einzeln abzugeben; eine Transporteinheit (120), die ein Reagenzglas von der Kassetteneinheit (110) aufnimmt und das Reagenzglas transportiert; eine Etikettendruckeinheit (140), die Informationen auf ein am Reagenzglas anzubringendes Etikett druckt; eine Etikettenanbringungseinheit (150), die das Reagenzglas von der Transporteinheit (120) empfängt, das mit Informationen bedruckte Etikett von der Etikettendruckeinheit (140) empfängt und das mit Informationen bedruckte Etikett am Reagenzglas anbringt; eine Reagenzglas-Aufbewahrungseinheit (160), die das mit einem Etikett versehene Reagenzglas aufnimmt, das von der Etiketten-Befestigungseinheit (150) empfangen wurde; und eine Steuerung (130), die operativ mit der Kassetteneinheit (110), der Transporteinheit (120), der Etikettendruckeinheit (140), der Etikettenanbringungseinheit (150) und der Reagenzglas-Aufbewahrungseinheit (160) verbunden ist, wobei die Etikettenanbringeinheit (150) einen internen Scanner (151) umfasst, der Barcodeinformationen des von der Etikettendruckeinheit (140) gedruckten Etiketts scannt, wobei die Steuerung (130) dazu konfiguriert ist: auf eine Datenbank zugreifen, die Identifikationsinformationen, Inspektionsinformationen und Inspektionsprioritätsinformationen eines Inspektionsziels speichert, steuern der Kassetteneinheit (110) und der Transporteinheit (120), um die Reagenzgläser auf der Grundlage der aus der Datenbank erhaltenen Inspektionsprioritätsinformationen nacheinander zu entladen und zur Etikettenanbringungseinheit (150) zu transportieren, und steuern der Etikettendruckeinheit (140), um Informationen, einschließlich der Identifikationsinformationen und der Inspektionsinformationen des Inspektionsziels, auf die Etiketten zu drucken, die an den Reagenzgläsern anzubringen sind, die nacheinander zur Etikettenanbringungseinheit (150) transportiert werden, und die Etikettenanbringungseinheit zu steuern (150) zum Anbringen der bedruckten Etiketten an den Reagenzgläsern, steuern des internen Scanners (151), um die Barcode-Informationen des am Reagenzglas angebrachten Etiketts zu scannen, um zu bestimmen, ob die Barcode-Informationen des von der Etikettenanbringungseinheit (150) am Reagenzglas angebrachten Etiketts mit den darin gespeicherten Barcode-Informationen übereinstimmen die Datenbank, generieren Sie eine Fehlermeldung basierend auf der Feststellung, dass die Barcode-Informationen des am Reagenzglas angebrachten Etiketts nicht mit den in der Datenbank gespeicherten Barcode-Informationen übereinstimmen, und wiederholen Sie den Vorgang des Entladens und Transportierens ein Reagenzglas an die Etikettenanbringeinheit (150), Drucken von Informationen durch die Etikettendruckeinheit (140) und Anbringen des gedruckten Etiketts durch die Etikettenanbringeinheit (150), wobei die Reagenzglas-Aufbewahrungseinheit (160) einen Sensor (161) zum Erkennen eines Zuflusses und eines Abflusses jedes Reagenzglases in der Reagenzglas-Aufbewahrungseinheit (160) umfasst, und wobei die Steuerung (130) als Reaktion auf den Empfang eines Zuflusserkennungssignals und eines Abflusserkennungssignals für ein Reagenzglas mit einer bestimmten Priorität vom Sensor (161) die Zuführung eines nächsten Reagenzglases mit der nächsten Priorität zur Etikettenanbringung ermöglicht Einheit (150) und ermöglicht auch das Drucken eines nächsten Etiketts mit Informationen einschließlich der Identifikationsinformationen und der Inspektionsinformationen des Inspektionsziels, das der nächsten Priorität entspricht, und ermöglicht das Anbringen des nächsten Etiketts am nächsten Reagenzglas.

2. Vorrichtung (100) zum Etikettieren von Reagenzgläsern nach Anspruch 1, weiterhin umfassend: ein Speichergerät einschließlich der Datenbank.

3. Vorrichtung (100) zum Etikettieren von Reagenzgläsern nach Anspruch 1, weiterhin umfassend: ein Kommunikationsmodul, das mit einem externen elektronischen Gerät kommunizieren kann, wobei die Datenbank im externen elektronischen Gerät gespeichert ist und der Controller (130) über das Kommunikationsmodul auf die Datenbank zugreift.

4. Computerlesbares Aufzeichnungsmedium, in dem ein Programm zum Ausführen von Vorgängen zum Anbringen eines Etiketts an einem Reagenzglas in der Reagenzglas-Etikettiervorrichtung (100) nach Anspruch 1 aufgezeichnet ist, wobei das Programm bei seiner Ausführung bewirkt, dass die Steuerung (130) des Reagenzglas-Etikettiergeräts (100), um: auf die Datenbank zugreifen, die Identifikationsinformationen, Inspektionsinformationen und Inspektionsprioritätsinformationen eines Inspektionsziels speichert, steuern der Kassetteneinheit (110) und der Transporteinheit (120) des Reagenzglas-Etikettiergeräts (100), um die Reagenzgläser nacheinander zu entladen und zur Etikettenanbringungseinheit (150) des Reagenzglas-Etikettiergeräts (100) zu transportieren über die aus der Datenbank erhaltenen Inspektionsprioritätsinformationen und steuern der Etikettendruckeinheit (140) der Reagenzglas-Etikettiervorrichtung (100), um Informationen, einschließlich der Identifikationsinformationen und der Inspektionsinformationen des Inspektionsziels, auf die Etiketten zu drucken, die an den Reagenzgläsern anzubringen sind, die nacheinander zur Etikettenanbringeinheit transportiert werden (150), steuern Sie die Etikettenanbringungseinheit (150), um die gedruckten Etiketten an den Reagenzgläsern anzubringen, und steuern Sie den internen Scanner (151), um die Barcode-Informationen des am Reagenzglas angebrachten Etiketts zu scannen, um festzustellen, ob die Barcode-Informationen des angebrachten Etiketts vorhanden sind an dem Reagenzglas, indem die Etikettenanbringungseinheit (150) mit einer in der Datenbank gespeicherten Barcode-Information übereinstimmt, generieren Sie eine Fehlermeldung basierend auf der Feststellung, dass die Barcode-Informationen des am Reagenzglas angebrachten Etiketts nicht mit den in der Datenbank gespeicherten Barcode-Informationen übereinstimmen, und wiederholen Sie den Vorgang des Entladens und Transportierens eines Reagenzglases an die Etikettenanbringungseinheit (150), Drucken von Informationen durch die Etikettendruckeinheit (140) und Anbringen des gedruckten Etiketts durch die Etikettenanbringungseinheit (150), wobei das Programm, wenn es ausgeführt wird, die Steuerung (130) der Reagenzglas-Etikettiervorrichtung (100) außerdem dazu veranlasst: als Reaktion auf den Empfang eines Zufluss-Detektionssignals und eines Abfluss-Detektionssignals für ein Reagenzglas mit einer bestimmten Priorität von dem Sensor (161), der in einer Reagenzglas-Aufbewahrungseinheit (160) der Reagenzglas-Etikettiervorrichtung (100) installiert ist, zur Erkennung einen Zufluss und einen Abfluss jedes Reagenzglases in der Reagenzglas-Aufbewahrungseinheit (160), ermöglichen die Zuführung eines nächsten Reagenzglases mit der nächsten Priorität zur Etikettenanbringungseinheit (150) und ermöglichen das Drucken eines nächsten Etiketts mit Informationen einschließlich der Identifikationsinformationen und der Inspektionsinformationen des Inspektionsziels entsprechend der nächsten Priorität und ermöglichen das Anbringen des nächsten Etiketts am nächsten Reagenzglas.

## Revendications

1. Appareil d'étiquetage de tubes à essai (100) comprenant: une unité de cassette (110) comprenant plusieurs cassettes (111), chaque cassette (111) étant configurée pour accueillir plusieurs tubes à essai et comprenant une sortie formée sur une partie inférieure afin d'évacuer les tubes à essai un par un; une unité de transport (120) recevant un tube à essai de l'unité de cassette (110) et transportant le tube à essai; une unité d'impression d'étiquettes (140) imprimant des informations sur une étiquette à attacher au tube à essai; une unité de fixation d'étiquettes (150) recevant le tube à essai de l'unité de transport (120), recevant l'étiquette imprimée par l'unité d'impression d'étiquettes (140) et fixant l'étiquette imprimée sur le tube à essai; une unité de stockage des tubes à essai (160) accueillant les tubes à essai étiquetés reçus de l'unité d'étiquetage (150); et un contrôleur (130) connecté de manière opérationnelle à l'unité de cassette (110), à l'unité de transport (120), à l'unité d'impression d'étiquettes (140), à l'unité de fixation d'étiquettes (150) et à l'unité de stockage des tubes à essai (160), dans lequel l'unité de fixation des étiquettes (150) comprend un scanner interne (151) qui lit les informations du code-barres de l'étiquette imprimée à partir de l'unité d'impression d'étiquettes (140), dans lequel le contrôleur (130) est configuré pour: accéder à une base de données qui stocke les informations d'identification, d'inspection et de priorité d'inspection d'une cible d'inspection, commander l'unité de cassette (110) et l'unité de transport (120) pour décharger et transporter séquentiellement les tubes à essai vers l'unité de fixation des étiquettes (150), sur la base des informations de priorité d'inspection obtenues à partir de la base de données, et commander l'unité d'impression d'étiquettes (140) pour imprimer des informations comprenant les informations d'identification et les informations d'inspection de la cible d'inspection sur les étiquettes à attacher aux tubes à essai transportés séquentiellement vers l'unité de fixation d'étiquettes (150), commander l'unité de fixation d'étiquettes (150) pour attacher les étiquettes imprimées aux tubes à essai, commander le scanner interne (151) pour scanner les informations du code-barres de l'étiquette attachée au tube à essai afin de déterminer si les informations du code-barres de l'étiquette attachée au tube à essai par l'unité de fixation d'étiquettes (150) correspondent aux informations du code-barres stockées dans la base de données, générer un message d'erreur après avoir déterminé que les informations du code-barres de l'étiquette attachée au tube à essai ne correspondent pas aux informations du code-barres stockées dans la base de données, et répéter l'opération de déchargement et de transport d'un tube à essai vers l'unité de fixation d'étiquettes (150), d'impression des informations par l'unité d'impression d'étiquettes (140) et de fixation de l'étiquette imprimée par l'unité de fixation d'étiquettes (150), dans lequel l'unité de stockage des tubes à essai (160) comprend un capteur (161) pour détecter une entrée et une sortie de chaque tube à essai dans l'unité de stockage des tubes à essai (160), et dans lequel, en réponse à la réception par le capteur (161) d'un signal de détection d'entrée et d'un signal de détection de sortie pour un tube à essai ayant une priorité particulière, le contrôleur (130) permet à un tube à essai suivant ayant une priorité suivante d'être fourni à l'unité de fixation d'étiquettes (150) et permet également à une étiquette suivante d'être imprimée avec des informations comprenant les informations d'identification et les informations d'inspection de la cible d'inspection correspondant à la priorité suivante et permet à l'étiquette suivante d'être attachée au tube à essai suivant.

2. Appareil d'étiquetage de tubes à essai (100) de la revendication 1, comprenant en outre: un dispositif de stockage comprenant la base de données.

3. Appareil d'étiquetage de tubes à essai (100) selon la revendication 1, comprenant en outre: un module de communication capable de communiquer avec un dispositif électronique externe, dans lequel la base de données est stockée dans le dispositif électronique externe, et le contrôleur (130) accède à la base de données par l'intermédiaire du module de communication.

4. Support d'enregistrement lisible par ordinateur dans lequel est enregistré un programme pour l'exécution des opérations de fixation d'une étiquette sur un tube à essai dans l'appareil d'étiquetage de tubes à essai (100) décrit dans la revendication 1, le programme amenant, lorsqu'il est exécuté, le contrôleur (130) de l'appareil d'étiquetage de tubes à essai (100) à: accéder à la base de données qui stocke les informations d'identification, d'inspection et de priorité d'inspection d'une cible d'inspection, commander l'unité de cassette (110) et l'unité de transport (120) de l'appareil d'étiquetage de tubes à essai (100) pour décharger et transporter séquentiellement les tubes à essai vers l'unité de fixation d'étiquettes (150) de l'appareil d'étiquetage de tubes à essai (100), sur la base des informations de priorité d'inspection obtenues à partir de la base de données, et commander l'unité d'impression d'étiquettes (140) de l'appareil d'étiquetage de tubes à essai (100) pour imprimer des informations comprenant les informations d'identification et les informations d'inspection de la cible d'inspection sur les étiquettes à attacher aux tubes à essai transportés séquentiellement vers l'unité de fixation d'étiquettes (150), commander l'unité de fixation des étiquettes (150) pour fixer les étiquettes imprimées aux tubes à essai, commander le scanner interne (151) pour scanner les informations du code-barres de l'étiquette fixée au tube à essai afin de déterminer si les informations du code-barres de l'étiquette fixée au tube à essai par l'unité de fixation des étiquettes (150) correspondent à des informations de code-barres stockées dans la base de données, générer un message d'erreur après avoir déterminé que les informations du code-barres de l'étiquette attachée au tube à essai ne correspondent pas aux informations du code-barres stockées dans la base de données, et répéter l'opération consistant à décharger et à transporter un tube à essai jusqu'à l'unité de fixation d'étiquettes (150), à imprimer des informations par l'unité d'impression d'étiquettes (140) et à attacher l'étiquette imprimée par l'unité de fixation d'étiquettes (150), dans lequel le programme, lorsqu'il est exécuté, fait en sorte que le contrôleur (130) de l'appareil d'étiquetage de tubes à essai (100): en réponse à la réception d'un signal de détection d'entrée et d'un signal de détection de sortie pour un tube à essai ayant une priorité particulière du capteur (161) qui est installé dans une unité de stockage de tubes à essai (160) de l'appareil d'étiquetage de tubes à essai (100) pour détecter une entrée et une sortie de chaque tube à essai dans l'unité de stockage du tube à essai (160), permettre à un tube à essai suivant ayant une priorité suivante d'être fourni à l'unité de fixation de l'étiquette (150), et permettre l'impression d'une étiquette suivante avec des informations comprenant les informations d'identification et les informations d'inspection de la cible d'inspection correspondant à la priorité suivante et permettre de fixer l'étiquette suivante au tube à essai suivant.
